(19) **Europäisches Patentamt / European Patent Office / Office européen des brevets**

(11) **EP 2 576 493 B1**

(12) **FASCICULE DE BREVET EUROPEEN**

(45) Date de publication et mention
de la délivrance du brevet:
**13.12.2017 Bulletin 2017/50**

(51) Int Cl.:
***C07C 67/08*** *(2006.01)*   ***C23F 14/02*** *(2006.01)*
***C23F 11/12*** *(2006.01)*

(21) Numéro de dépôt: **11729165.8**

(22) Date de dépôt: **19.05.2011**

(86) Numéro de dépôt international:
**PCT/IB2011/001078**

(87) Numéro de publication internationale:
**WO 2011/151691 (08.12.2011 Gazette 2011/49)**

(54) **COMPOSITION CATALYTIQUE A EFFET INHIBITEUR DE CORROSION, PROCEDE D'INHIBITION DE LA CORROSION ET SON UTILISATION**

KATALYTISCHE ZUSAMMENSETZUNG MIT KORROSIONSHEMMENDER WIRKUNG, VERFAHREN ZUR KORROSIONSHEMMUNG UND VERWENDUNG

CATALYTIC COMPOSITION HAVING A CORROSION-INHIBITING EFFECT, METHOD FOR INHIBITING CORROSION AND USE OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.06.2010 FR 1054364**

(43) Date de publication de la demande:
**10.04.2013 Bulletin 2013/15**

(73) Titulaire: **Rhodia Poliamida E Especialidades Ltda Sao Paulo - SP (BR)**

(72) Inventeurs:
• **MARENCO, Carlos, Eduardo**
  **CEP-13105-800 Campinas, SP (BR)**
• **LOURENCO, Wagner**
  **CEP-13083-130 Campinas, SP (BR)**
• **BRESCIANI, Renato**
  **CEP-13083-130 Campinas, SP (BR)**

• **MARTINS, Sergio**
  **CEP-13083-000 Cidade Universitaria (BR)**
• **PATTARO, Mario**
  **CEP-13950-543 Campinas, SP (BR)**
• **BONIFACIO, Wellington**
  **CEP-13085-220 Barão Geraldo (BR)**
• **LAZARINI, João Carlos**
  **CEP-13083-830 Campinas (BR)**

(74) Mandataire: **Ridray, Annabelle et al
Rhodia Opérations
85, avenue des Frères Perret - BP 62
69192 Saint-Fons Cedex (FR)**

(56) Documents cités:
EP-A1- 0 066 059   EP-A1- 1 253 113
EP-A1- 2 060 555   EP-A2- 0 158 499
US-A- 4 365 081   US-A1- 2009 012 324

**Description**

La présente invention concerne une composition catalytique à effet inhibiteur de corrosion des surfaces métalliques en contact avec un milieu réactionnel d'estérification comprenant un acide carboxylique, un alcool et un catalyseur acide.

L'invention vise également un procédé d'inhibition de la corrosion de telles surfaces métalliques ainsi que l'utilisation d'un composé spécifique comme inhibiteur de corrosion dans une réaction d'estérification entre un acide carboxylique et un alcool, en présence d'un catalyseur acide.

Les procédés d'estérification, et notamment ceux de fabrication de l'acétate d'éthyle doivent être les plus performants possibles, en termes de productivité, et conduire à un produit de qualité.

En effet, l'acétate d'éthyle étant un produit de consommation courante, à gros volume, fabriqué dans des installations industrielles, les enjeux économiques relatifs à sa production sont significatifs.

Les procédés classiques de fabrication de l'acétate d'éthyle utilisent des catalyseurs acides, souvent de l'acide sulfurique lorsqu'il s'agit de mettre en oeuvre une étape d'estérification couplée à une étape de distillation, comme décrit par exemple dans le brevet GB1173089. Il peut également s'agir de résines sulfoniques lorsque ces procédés mettent en oeuvre une distillation réactive telle que décrite dans la demande EP1220829.

Cependant, la présence de l'acide acétique, du catalyseur acide ainsi que d'impuretés formées en cours de réaction (telles que par exemple des sels de sulfates) dans de tels procédés fonctionnant à des températures relativement élevées (de l'ordre de 100°C et supérieures) est la cause de phénomènes corrosifs importants.

Ces phénomènes se traduisent par une dégradation et un encrassement des parois des équipements en contact avec ces acides. Par « encrassement », on entend que des sels, en particuliers des sels de fer, de nickel et de chrome, issus du processus corrosif se sont déposés sur les parois. Il s'agit notamment des parois du réacteur, de la colonne de distillation réactive, des rebouilleurs, des tuyauteries, pompes et vannes. Pour les parois des équipements tels que le rebouilleur, ces phénomènes entraînent une réduction de la surface d'échange et du coefficient global de transfert de chaleur du rebouilleur. De tels phénomènes nécessitent donc des arrêts de productions fréquents pour maintenance des équipements. Il s'agit notamment de remplacer ou de traiter les surfaces corrodées ou encrassées. Ces interruptions fréquentes dans la fabrication de l'acétate d'éthyle sont autant de pertes en termes de productivité, de capacité de production de l'installation et en coût d'équipement pour les fabricants. En effet, d'une part, l'encrassement des parois des rebouilleurs conduit à une baisse de productivité en acétate d'éthyle puisque le coefficient d'échange entre la vapeur chauffant le réacteur et le milieu réactionnel est diminué. D'autre part, les arrêts fréquents sont autant de temps pendant lequel l'unité ne produit pas d'acétate d'éthyle.

Il est connu d'utiliser des réacteurs en matériaux réputés inoxydables mais ces équipements sont onéreux ou insuffisamment résistants à la corrosion de l'acide acétique et du catalyseur acide.

Il est également connu d'utiliser des inhibiteurs de corrosion pour réduire les phénomènes corrosifs. Il peut s'agir notamment d'utiliser des cations métalliques oxydants comme le cuivre ou le fer sous forme de sulfates, notamment dans milieux comprenant des acides oxydants comme l'acide sulfurique. Ceci étant, de tels composés ne sont pas suffisamment efficaces pour éviter la corrosion des équipements dans des milieux comprenant de l'acide acétique et un catalyseur acide, surtout à des températures supérieures à 100°C, et les phénomènes corrosifs sont toujours observés. EP0158499 et EP2060555 divulguent que afin d'éviter la corrosion de la surface métallique du réacteur d'estérification par le catalyseur acide, 0,1 à 1 % en masse d'inhibiteur de corrosion, par exemple de l'acétate de cuivre, est ajoutée au milieu d'estérification.

L'objectif de la présente invention est donc de pallier les inconvénients sus évoqués liés à la corrosion des installations par les acides carboxyliques et catalyseurs acides mis en jeu lors d'une réaction d'estérification entre un acide carboxylique et un alcool, en présence d'un catalyseur acide.

Dans ce but, la présente invention fournit une composition, notamment une composition catalytique à effet inhibiteur de corrosion des surfaces métalliques en contact avec un milieu d'estérification comprenant un acide carboxylique et un alcool, caractérisée par le fait qu'elle comprend au moins, à titre de catalyseur, un acide protonique fort, c'est-à-dire présentant un pKa dans l'eau inférieur à 2, et, à titre d'inhibiteur de corrosion, un carboxylate de fer (III).

Un autre objet de l'invention est un procédé pour inhiber la corrosion des surfaces métalliques en contact avec un milieu d'estérification comprenant un acide carboxylique, un alcool, et un acide protonique fort, c'est-à-dire présentant un pKa dans l'eau inférieur à 2, comme catalyseur, caractérisé par le fait que l'on met en oeuvre, lors d'une réaction d'estérification entre ledit acide carboxylique et ledit alcool en présence dudit acide protonique fort, un carboxylate de fer (III), correspondant de préférence à l'acide carboxylique à estérifier.

Un autre objet de l'invention est l'utilisation d'un carboxylate de fer (III) comme inhibiteur de corrosion des surfaces métalliques en contact avec un milieu d'estérification comprenant un acide carboxylique, un alcool et un catalyseur acide protonique fort, c'est-à-dire présentant un pKa dans l'eau inférieur à 2.

Un autre objet de l'invention est une composition comprenant au moins un acide protonique fort choisi parmi l'acide para-toluènesulfonique ou l'acide méthanesulfonique et un carboxylate de fer (III).

La composition, le procédé et l'utilisation selon la présente invention permettent en effet de limiter considérablement les phénomènes de corrosion et d'encrassement par les acides carboxyliques et catalyseurs acides mis en jeu lors de la réaction d'estérification et ainsi d'éviter les arrêts de production pour remplacement ou nettoyage des surfaces encrassées. Ainsi, la présente invention permet à la fois un gain de productivité dû au maintien d'un bon coefficient d'échange entre la vapeur et le milieu devant être chauffé au cours du temps, un gain économique relatif à la durée de vie plus longue des équipements, ainsi qu'une capacité de production maximum de l'installation ne nécessitant plus d'arrêts de production liés à la corrosion. Par « inhibition de la corrosion » on entend que la vitesse de corrosion doit être inférieure à 0,125 mm/an, de préférence inférieure à 0,05 mm/an et encore plus préférentiellement inférieure à 0,01 mm/an.

Les modes de réalisations particuliers de l'invention ci-dessous décrits s'appliquent aussi bien à la composition, au procédé et à l'utilisation selon l'invention.

Conformément à un mode de réalisation de l'invention, le carboxylate de fer (III) est celui correspondant à l'acide carboxylique à estérifier, de préférence l'acétate de fer (III). Le carboxylate de fer (III) est avantageusement un composé soluble dans le milieu d'estérification.

L'acide carboxylique à estérifier est avantageusement choisi parmi les acides carboxyliques aliphatiques, ayant de 1 à 6 atomes de carbone, de préférence il s'agit de l'acide acétique.

L'acide carboxylique à estérifier est avantageusement introduit pur ou en solution aqueuse très concentrée. L'invention n'exclut pas la présence d'eau dans l'acide carboxylique. Cependant, il est préférable d'utiliser de l'acide carboxylique pur du fait de la nécessité ultérieure d'éliminer l'eau présente dans l'ester d'acide carboxylique obtenu.

L'alcool mis en jeu est de préférence un alcool à chaîne alkyle linéaire ou ramifiée ayant de 1 à 6 atomes de carbone ou un alcool à chaîne cycloalkyle ayant 5 ou 6 atomes de carbone.

On préfère notamment les alcools à bas point d'ébullition, notamment inférieur à 170°C, de préférence inférieur à 165°C.

Ainsi, l'alcool à chaîne alkyle est avantageusement choisi parmi l'éthanol, le n-propanol, le butanol, de préférence l'éthanol.

L'alcool à chaîne cycloalkyle est de préférence le cyclohexanol.

Un excès de l'un des réactifs peut être avantageux pour déplacer l'équilibre de la réaction vers la production d'ester d'acide carboxylique.

Ainsi le ratio molaire entre l'acide carboxylique et l'alcool peut être compris entre 0,9 et 25, de préférence entre 1 et 20.

On préfère généralement un excès d'acide carboxylique par rapport à l'alcool, de préférence avec un ratio molaire entre l'acide carboxylique et l'alcool compris entre 7 et 25, notamment entre 9 et 20.

La borne supérieure, pour des raisons économiques, est avantageusement choisie inférieure à 25. Le ratio précisément défini correspond au ratio molaire des réactifs en début de réaction.

Le catalyseur intervenant dans l'invention est un catalyseur acide protonique fort, c'est-à-dire présentant un pKa dans l'eau inférieur à 2. Il peut s'agir d'un catalyseur acide liquide (ou soluble dans le milieu réactionnel) permettant une catalyse homogène ou un catalyseur acide solide (insoluble dans le milieu réactionnel) permettant une catalyse hétérogène.

Selon un premier mode, le catalyseur est un catalyseur acide liquide permettant une catalyse homogène.

Par acide protonique fort, on désigne dans la présente invention, un acide présentant un pKa dans l'eau inférieur à 2, de préférence inférieur à 1.

Le pKa est défini comme suit pKa = - log Ka, Ka étant la constante de dissociation ionique du couple acide/base à température ambiante (généralement 25°C), lorsque l'eau est utilisée comme solvant.

Parmi les acides répondant à cette définition, il est préférable d'utiliser un acide ne conduisant pas à des réactions parasites gênantes pour le procédé d'estérification et en particulier ne présentant pas de caractère oxydant, comme l'acide nitrique.

On peut citer comme acide protonique fort plus particulièrement l'acide sulfurique, les acides sulfoniques et leurs mélanges.

Comme acides sulfoniques, on peut mentionner notamment l'acide fluorosulfonique, l'acide chlorosulfonique ou l'acide trifluorométhanesulfonique, l'acide méthanesulfonique, l'acide éthanesulfonique, l'acide camphène-sulfonique, l'acide benzènesulfonique, les acides toluènesulfoniques, les acides xylènesulfoniques, les acides naphtalènesulfoniques.

Parmi ces acides, le catalyseur préféré est choisi parmi l'acide para-toluènesulfonique ou l'acide méthanesulfonique, de préférence l'acide méthanesulfonique.

Selon un second mode, le catalyseur est un catalyseur acide solide conduisant à une catalyse hétérogène.

Les catalyseurs acides solides de l'invention sont préférentiellement des résines sulfoniques ou des zéolithes.

Les zéolithes qui peuvent être utilisées sont par exemple celles citées dans le document WO 2007/099071.

Les résines qui conviennent à la présente invention peuvent être constituées d'un squelette polystyrénique ou polyacrylique qui porte des groupes fonctionnels sulfoniques ou carboxyliques.

Ainsi, on peut mettre en oeuvre les résines acides sulfoniques $SO_3H$ ou carboxyliques COOH existant sur le marché, résines commercialisées sous différentes dénominations commerciales.

On peut citer, entre autres, les résines d'estérification suivantes : Amberlyst® 15 de Rohm Haas, Amberlite® IR-120H de Rohm Haas, Lewatit® 2631 de Bayer et K1431 de Bayer.

L'acidité de ces résines est par exemple comprise entre 1 et 10 eq/kg (H+).

Ces résines sont notamment mises en oeuvre en lit fixe ou fluidisé, de préférence en lit fixe.

La quantité de catalyseur acide protonique fort introduite est de préférence comprise entre 0,05 et 15 % en masse, de préférence de 0,1 à 10 % en masse et notamment de 0,2 à 2 % en masse par rapport à la masse de milieu réactionnel en début de réaction.

Selon l'invention, la réaction d'estérification est conduite de préférence dans des conditions telles le mélange réactionnel est à l'état liquide.

Selon un premier mode de réalisation de l'invention, la réaction d'estérification est mise en oeuvre dans un procédé en deux étapes : une réaction d'estérification suivie d'une opération de distillation. La réaction d'estérification peut être conduite en catalyse homogène ou hétérogène, de préférence en catalyse homogène.

La réaction d'estérification est de préférence conduite dans un réacteur d'estérification.

Conformément à l'invention, la réaction d'estérification peut être conduite selon un mode continu ou discontinu, de préférence en continu.

Dans le réacteur d'estérification, l'acide carboxylique et l'alcool peuvent être introduits seuls ou en mélange, de préférence en mélange.

Selon un mode de réalisation de l'invention, la réaction d'estérification est mise en oeuvre à une température au moins égale à 50°C.

De façon avantageuse, la température de la réaction est comprise entre 50 et 150°C, de préférence entre 100 et 130°C.

Selon l'invention, la réaction d'estérification est de préférence conduite à une pression telle le mélange réactionnel est à l'état liquide.

La réaction est préférentiellement conduite à pression atmosphérique. Une pression légèrement supérieure ou inférieure à la pression atmosphérique peut également convenir. Ainsi, la réaction d'estérification peut être mise en oeuvre, par exemple, à une pression absolue comprise entre 0,5 et 5 bars absolus.

Le mélange réactionnel ainsi obtenu en sortie de réaction d'estérification est ensuite soumis à une opération de distillation afin d'obtenir en tête de distillation un flux vapeur comprenant majoritairement l'ester d'acide carboxylique et minoritairement l'alcool, de l'eau et des traces d'acide carboxylique.

Dans le présent texte, on entend par « flux comprenant majoritairement un composé donné », que le composé donné représente au moins à 85 % de la masse du flux, de préférence au moins 90 % de la masse du flux.

Par « flux comprenant minoritairement un composé donné », on entend que le composé donné représente moins de 15 % de la masse du flux, de préférence moins de 10 % de la masse du flux.

Par « traces », on entend moins de 0,02 % en masse, notamment entre 0,01 et 0,02 % en masse du composé concerné.

L'opération de distillation est de préférence conduite dans une colonne de distillation. Le point d'alimentation où est introduit le mélange réactionnel est en général sensiblement à mi-hauteur de la colonne de distillation. Il peut être également situé plus bas et à une hauteur comprise entre la mi-hauteur et le pied de la colonne.

La température en pied de distillation est de préférence comprise entre 50 et 150°C, de préférence entre 100 et 130°C.

La pression définie en tête de distillation est de préférence comprise entre 0,5 et 5 bars absolus, avantageusement la pression en tête de distillation est comprise entre 1 et 2 bars absolus.

Selon ce premier mode de réalisation de l'invention dans lequel la réaction d'estérification est mise en oeuvre dans un procédé en deux étapes, réaction puis distillation, de préférence dans un réacteur d'estérification couplé à une colonne de distillation, le carboxylate de fer (III) est introduit avec les réactifs avant leur introduction dans le réacteur ou bien dans le réacteur avant l'introduction des réactifs.

Dans le cas d'une catalyse homogène, le catalyseur acide liquide permettant une catalyse homogène est introduit avec les réactifs avant leur introduction dans le réacteur ou bien dans le réacteur avant l'introduction des réactifs. Il est possible de réaliser une ou plusieurs purges du réacteur en cours de réaction afin de débarrasser le milieu réactionnel de certaines impuretés lourdes qui s'accumulent dans le réacteur. Dans ce cas, une partie du carboxylate de fer (III) et du catalyseur acide liquide est éliminée. Dans ce cas, du carboxylate de fer (III) et du catalyseur acide protonique fort peuvent être rajoutés, ensemble ou séparément, en continu ou ponctuellement, dans le réacteur au cours de la réaction.

Dans le cas d'une catalyse hétérogène, le catalyseur acide solide est placé en lit fixe ou fluidisé dans le réacteur et le carboxylate de fer (III) est introduit dans la colonne de distillation lors de l'étape de distillation. De manière préférée, le carboxylate de fer (III) est introduit en pied de colonne de distillation. Selon un mode préféré de l'invention, le carboxylate de fer (III) n'est pas en contact avec le catalyseur solide permettant une catalyse hétérogène.

Selon ce premier mode avantageux de réalisation de l'invention (procédé en deux étapes), le carboxylate de fer (III) est introduit dans une concentration allant de 300 à 1500 ppm dans le milieu réactionnel, de préférence entre 400 et 1200 ppm.

Par « milieu réactionnel » on entend le milieu comprenant l'acide carboxylique, l'alcool, l'ester d'acide carboxylique et éventuellement le catalyseur acide protonique fort lorsqu'il s'agit d'un catalyseur acide liquide permettant une catalyse homogène.

Selon le mode particulier de réalisation de l'invention dans lequel la réaction d'estérification est conduite en présence d'un excès d'acide carboxylique, le milieu en pied de distillation est avantageusement soutiré et recyclé par réintroduction en amont ou au cours de la réaction d'estérification, de préférence en amont.

Par « en amont » on entend que le milieu en pied de distillation est réintroduit dans le mélange d'acide carboxylique et d'alcool avant qu'il ait réagi.

Dans ce cas, le ratio entre le débit d'alimentation du mélange réactionnel (acide carboxylique + alcool + ester d'acide carboxylique + éventuellement le catalyseur homogène) et le débit du flux de recyclé est avantageusement compris entre 4 et 20, de préférence entre 5 et 15.

Selon un second mode de réalisation de l'invention, la réaction d'estérification est mise en oeuvre dans un procédé en une étape, cette étape consistant en une opération de distillation réactive. L'opération de distillation réactive a lieu par exemple dans une colonne de distillation réactive.

Selon ce mode particulier selon lequel la réaction d'estérification et la distillation sont conduites en une seule et même étape, on obtient également en tête de distillation un flux vapeur comprenant majoritairement l'ester d'acide carboxylique et minoritairement l'alcool, de l'eau et des traces d'acide carboxylique.

Les définitions des termes « majoritairement », « minoritairement » et « traces » sont identiques à celles données précédemment.

La distillation réactive peut être conduite en catalyse homogène ou hétérogène, de préférence en catalyse hétérogène.

Dans le cas d'une catalyse homogène, le catalyseur acide liquide permettant une catalyse homogène est introduit avec les réactifs avant leur introduction dans la colonne ou bien en pied de colonne avant l'introduction des réactifs.

De façon générale, le composé le plus volatil (entre l'acide carboxylique et l'alcool) est alimenté dans la partie inférieure de la colonne et l'autre est alimenté dans la partie supérieure de la colonne. Généralement, c'est l'alcool qui est le composé le plus volatil. L'alcool est alors préférentiellement alimenté dans la partie inférieure de la colonne et l'acide carboxylique est préférentiellement alimenté dans la partie supérieure de la colonne.

On préfère tout particulièrement que le rapport molaire acide carboxylique / alcool soit compris entre 1 et 2, de préférence entre 1 et 1,5.

La température en pied de distillation réactive est de préférence comprise entre 50 et 150°C, de préférence entre 90 et 130°C.

Selon l'invention, la réaction d'estérification par distillation réactive est de préférence conduite à une pression telle le mélange réactionnel est à l'état liquide.

La pression définie en tête de distillation réactive est de préférence comprise entre 0,5 et 5 bars absolus, avantageusement la pression en tête de distillation est comprise entre 1 et 2 bars absolus.

Pour la distillation réactive en catalyse homogène, qui a lieu par exemple dans une colonne de distillation réactive, le carboxylate de fer (III) est introduit avec les réactifs avant leur introduction dans la colonne ou bien en pied de colonne avant ou après l'introduction des réactifs.

Dans le cas d'une catalyse hétérogène, la colonne de distillation réactive comprend notamment au moins une zone réactionnelle et au moins une zone non réactionnelle.

Le corps de garnissage qui peut être utilisé dans la colonne de distillation réactive est choisi en fonction de l'efficacité nécessaire. Le corps de garnissage peut être choisi parmi les corps de garnissage bien connus de l'homme du métier, tels que par exemple des solides sous forme d'anneaux, d'extrudés polylobés ou de selles. A titre d'exemple non limitatif de corps de garnissage, on peut citer les anneaux de Raschig, les anneaux de Pall, les anneaux d'Intos, les selles de Berl, les selles Novalox et les selles Intalox. Mais le corps de garnissage peut aussi être choisi parmi les garnissages structurés, par exemple de type FLEXIPAC (marque déposée) commercialisés par la société Koch, ou SULZER CHEMTECH ou SULZER (marques déposées) commercialisés par la société Sulzer.

La zone réactionnelle de la colonne de distillation réactive comprend généralement au moins un lit catalytique d'estérification (catalyseur acide solide tel que défini ci-dessus). La zone non-réactionnelle correspond à la zone dépourvue de catalyseur, comprenant généralement un garnissage ordonné, vrac ou plateaux.

Le catalyseur peut être enfermé dans au moins une enveloppe perméable, enveloppe constituée par exemple par une toile en tissu, en matériau synthétique par exemple en polypropylène, ou en tissu métallique.

Le catalyseur peut également être disposé en vrac, c'est-à-dire librement, à l'intérieur de chaque lit catalytique de la zone catalytique.

Dans ce cas, pour maintenir le catalyseur en place et pour éviter qu'il soit entraîné par le courant de liquide qui le traverse, on prévoit généralement que tout lit catalytique compris dans la zone catalytique repose sur tout dispositif permettant le passage du liquide mais imperméable aux particules catalytiques, tel que par exemple une grille.

Lorsque le catalyseur solide est disposé en vrac, on peut prévoir de l'utiliser sous forme de lit fixe, de lit expansé ou de lit fluidisé. Que le catalyseur soit disposé en vrac ou enfermé dans au moins une enveloppe, le taux de vide que l'on peut lui conférer est compris généralement entre 30 et 70%.

Dans la colonne de distillation réactive, les étapes suivantes sont généralement mises en oeuvre:

1) l'alcool et l'acide carboxylique sont alimentés dans une colonne de distillation comprenant au moins une zone réactionnelle et au moins une zone non réactionnelle ;

2) l'alcool et l'acide carboxylique réagissent dans la ou les zones réactionnelles en présence d'un catalyseur solide permettant une catalyse hétérogène, et les composés formés sont séparés par distillation.

De façon générale, le composé le plus volatil (entre l'acide carboxylique et l'alcool) est alimenté dans la partie inférieure de chaque zone réactionnelle et l'autre est alimenté dans la partie supérieure de chaque zone réactionnelle. Généralement, c'est l'alcool qui est le composé le plus volatil. L'alcool est alors préférentiellement alimenté dans la partie inférieure de chaque zone réactionnelle ou en dessous de chacune de ces zones et l'acide carboxylique est préférentiellement alimenté dans la partie supérieure de chaque zone réactionnelle ou au dessus de chacune de ces zones.

On préfère tout particulièrement que la réaction à l'étape 2) soit effectuée de manière à ce que le rapport molaire acide carboxylique / alcool soit compris entre 1 et 2, de préférence entre 1 et 1,5.

La proportion en catalyseur solide de catalyse hétérogène dans les sections réactives est préférentiellement comprise entre 10 et 50 % en volume.

La température en pied de distillation réactive est de préférence comprise entre 50 et 150°C, de préférence entre 90 et 130°C.

La pression définie en tête de distillation réactive est de préférence comprise entre 0,5 et 5 bars absolus, avantageusement la pression en tête de distillation est comprise entre 1 et 2 bars absolus.

Pour la distillation réactive en catalyse hétérogène, qui a lieu par exemple dans une colonne de distillation réactive, le carboxylate de fer (III) est introduit avec le composé le plus volatil (entre l'acide carboxylique et l'alcool) qui est alimenté dans la partie inférieure de la zone réactionnelle ou bien en pied de colonne avant ou après l'introduction des réactifs. Dans le cas où il y a plusieurs zones réactionnelles, le carboxylate de fer (III) est introduit en dessous de l'ensemble des zones réactionnelles ou bien en pied de colonne avant ou après l'introduction des réactifs. Selon un mode préféré de l'invention, le carboxylate de fer (III) n'est pas en contact avec le catalyseur solide permettant une catalyse hétérogène.

Dans ce cas notamment, du carboxylate de fer (III) peut être rajouté, en continu ou ponctuellement, dans la partie inférieure de chaque zone réactionnelle ou bien en pied de colonne au cours de la distillation réactive.

Selon ce second mode avantageux de réalisation de l'invention (procédé en une étape), le carboxylate de fer (III) est introduit dans une concentration allant de 300 à 1500 ppm dans le milieu réactionnel, de préférence entre 400 et 1200 ppm. Par « milieu réactionnel » on entend le milieu comprenant l'acide carboxylique, l'alcool, l'ester d'acide carboxylique et éventuellement le catalyseur acide protonique fort lorsqu'il s'agit d'un catalyseur acide liquide permettant une catalyse homogène.

Selon l'invention, les surfaces métalliques en contact avec le milieu d'estérification comprenant un acide carboxylique, un alcool et un catalyseur acide protonique fort sont des surfaces susceptibles de résister à la corrosion du milieu réactionnel.

A cet effet, on choisit des matériaux pour la partie en contact avec le milieu réactionnel résistant à la corrosion comme les alliages au nickel pouvant contenir du molybdène, du chrome, du cobalt, du fer, du cuivre, du manganèse, du titane, du zirconium, de l'aluminium, du carbone et du tungstène, par exemple vendus sous les marques HASTELLOY®, principalement le HASTELLOY C-22 (UNS N06022).

On peut choisir également les aciers inoxydables, tels que les aciers austénitiques [Robert H. Perry et al, Perry's Chemical Engineers' Handbook, Sixth Edition (1984), page 23-44]. et plus particulièrement les aciers inoxydables du type 304, 304 L, 316 ou 316 L. On met en oeuvre un acier ayant une teneur en nickel au plus de 22 % en masse, de préférence comprise entre 6 et 20 %, et plus préférentiellement comprise entre 8 et 14 %.

Les aciers inoxydables 304 et 304 L ont une teneur en nickel variant entre 8 et 12% et les aciers 316 et 316 L ont une teneur en nickel variant entre 10 et 14%.

On fait appel plus particulièrement aux aciers inoxydables 316 L.

On peut choisir également les aciers inoxydables austéno-ferritique dit duplex de type X2CrNiMoN22-5-3 (1.4462) ou X2CrNiMoN25-7-4 (1.4410) selon la nomenclature européenne.

Les exemples qui suivent, illustrent l'invention sans toutefois la limiter.

Exemples

Exemple 1 - Acide p-toluène sulfonique (APTS) comme catalyseur et fer III comme inhibiteur de corrosion - effet de la nature de l'inhibiteur et de la température :

On va tester l'efficacité des différents inhibiteurs de corrosion en effectuant un test qui consiste dans un réacteur fermé (acier inoxydable en acier 316L avec revêtement en PTFE (polytétrafluoroéthylène)) à immerger des éprouvettes en acier inoxydable de type 316L préalablement nettoyées, séchées et pesées sont plongées, dans un milieu réactionnel issu d'une réaction d'estérification entre l'acide acétique et l'éthanol en présence d'un catalyseur acide permettant une catalyse homogène, puis à quantifier la vitesse de corrosion.

Ces tests ont été conduits selon les normes ASTM G1 « Pratice for preparing, cleaning, and evaluating corrosion test spécimens » et ASTM G31 « Pratice for laboratory immersion corrosion testing of metals ».

On reconstitue le milieu réactionnel issu d'une réaction d'estérification entre de l'acide acétique et de l'éthanol en présence d'APTS. Le milieu réactionnel a la composition suivante :

- acide acétique 75,5% ;
- éthanol 8% ;
- acétate d'éthyle 9,2% ;
- eau 6% ;
- APTS 1 % ;
- bisulfate de sodium 0,2% ;
- acide formique 0,1 % ;

Le bisulfate de sodium est introduit de façon à représenter le milieu corrosif dans les installations industrielles où les impuretés présentes dans le catalyseur génèrent notamment des sels de sulfate, qui sont très corrosifs vis-à-vis des métaux.

A partir de ce milieu réactionnel, on teste les différents inhibiteurs de corrosion à base de fer (III) en ajoutant, selon les essais, un inhibiteur choisi parmi:

- le nitrate de fer III $Fe(NO_3)_3$ ;
- le sulfate de fer III $Fe(SO_4)_3$, et
- la limaille de fer, qui, en présence d'acide acétique génère de l'acétate de fer III (on ajoute l'équivalent de 1000 ppm de fer III).

Le milieu réactionnel est introduit dans le réacteur et chauffé à une température T de 110°C ou 140°C selon les tests. Toutes les 48 heures, le milieu réactionnel est renouvelé pour maintenir une quantité constante d'agents corrosifs.

Après 350 heures, on retire les éprouvettes, que l'on les nettoie, sèche et pèse. On mesure ensuite la vitesse de corrosion pour les différents tests (avec et sans chacun des inhibiteurs ci-dessus listés).

La vitesse de corrosion est calculée comme suit :

$$Vc = 8{,}76 \times 10^4 . \Delta P / A . T . D$$

avec

(Vc) = vitesse corrosion (mm/an)
$\Delta P$ = variation de masse (g) ;
A = surface ($cm^2$) ;
T = temps (heures) ;
D = densité ($g/cm^3$).

Les résultats sont réunis ci-dessous dans le tableau 1.

Tableau 1

| | Vitesse Corrosion (mm/an) | | | |
|---|---|---|---|---|
| | Sans Inhibiteur | Fe(NO$_3$)$_3$ | Fe(SO$_4$)$_3$ | Acétate de Fe III |
| **110°C** | 0,30 | <0,01 | < 0,01 | < 0,01 |
| **140°C** | 0,40 | < 0,01 | 0,30 | < 0,01 |

Les résultats montrent l'efficacité du nitrate et de l'acétate de fer III, dont la vitesse de corrosion à 140°C est passée de 0,40 mm/an à <0,01 mm/an dans le cas du nitrate et de l'acétate de fer III. La valeur « <0,01 mm/an » est due à l'incertitude sur la mesure qui ne permet pas l'obtention d'une valeur plus précise de l'ordre du millième.

Cependant, le nitrate de fer III ne peut pas être utilisé comme inhibiteur de corrosion dans la synthèse de l'acétate d'éthyle puisqu'il contamine l'acétate d'éthyle. Il est en outre dangereux de l'utiliser du fait qu'il génère des oxydes d'azote (NOx).

Dans le cas du sulfate de fer III, qui est efficace à 110°C (vitesse corrosion inférieure à 0,01 mm/an). Cependant, on n'observe plus aucune action sur la vitesse de corrosion à 140°C, qui se maintient à 0,30 mm/an avec ledit inhibiteur.

Exemple 2 - Essais comparatifs entre l'acide p-toluène sulfonique (APTS) et l'acide méthane sulfonique (AMS) avec et sans inhibiteur de corrosion acétate de fer III :

On va tester l'efficacité de l'inhibiteur de corrosion acétate de fer (III) en effectuant un test qui consiste dans un réacteur fermé (acier inoxydable en acier 316L avec revêtement en PTFE (polytétrafluoroéthylène) à immerger des éprouvettes en acier inoxydable de type 316L préalablement nettoyées, séchées et pesées sont plongées, dans un milieu réactionnel issu d'une réaction d'estérification entre l'acide acétique et l'éthanol en présence de différents catalyseurs acide permettant une catalyse homogène, puis à quantifier la vitesse de corrosion.

On compare ainsi l'efficacité dans l'effet inhibiteur de corrosion en fonction du catalyseur utilisé (APTS ou AMS).

Les tests ont été réalisés dans des conditions très corrosives (140°C) représentatives des conditions subies lors du procédé industriel de fabrication de l'acétate d'éthyle par estérification en présence d'acide acétique et d'éthanol. On comparera les vitesses de corrosion selon le catalyseur acide utilisé et la présence ou non de l'inhibiteur de corrosion acétate de fer (III), sur les principaux matériaux utilisés, c'est-à-dire de l'acier inoxydable austénitique 316 L et duplex type X2CrNiMoN22-5-3 (1.4462).

On reconstitue le milieu réactionnel issu d'une réaction d'estérification entre de l'acide acétique et de l'éthanol en présence d'un catalyseur acide.

Le milieu réactionnel a la composition suivante :

- acide acétique 75,5 % ;
- éthanol 8,0 % ;
- acétate d'éthyle 9,7 % ;
- eau 6,0%
- acide formique 0,1 % ;
- bisulfate de sodium 0,2 % ;
- APTS 0,5% ou AMS 0,28% ou AMS 0,8%.

Le bisulfate de sodium est introduit pour les mêmes raisons que celles invoquées supra pour l'exemple 1.

Selon les essais on choisit les catalyseurs suivants APTS à 0,5 %, AMS à 0,28 % ou AMS à 0,8 % et l'on ajoute ou non (selon les cas) de l'acétate de fer (III) comme inhibiteur de corrosion, l'acétate de fer III est mis en oeuvre dans une concentration telle qu'elle correspond à 1000 ppm (0,1%) de fer III dans le milieu réactionnel.

Après avoir placé le milieu réactionnel dans le réacteur fermé (cf. exemple 1) contenant les éprouvettes préalablement nettoyées, séchées et pesées, on chauffe à une température de 140°C pendant 350 heures avec renouvellement du milieu réactionnel toutes les 48h comme pour l'exemple 1.

On retire ensuite les éprouvettes du réacteur puis l'on procède au nettoyage, séchage et à la pesée des éprouvettes.

Le calcul de la vitesse de corrosion est réalisé grâce à la formule donnée plus haut pour l'exemple 1. Les résultats sont rassemblés dans le tableau 2 ci-dessous.

Tableau 2

| MATERIAU | COMPOSITION CATALYTIQUE (T = 140°C) | VITESSE DE CORROSION (mm/an) |
|---|---|---|
| AISI 316 L | APTS 0,5% | 0,16 à 0,26 |
| | APTS 0,5% + AcOFe(III) 0,1% | <0,01 |
| | AMS 0,28% | 0,23 à 0,33 |
| | AMS 0,28% + AcOFe(III) 0,1% | <0,01 |
| | AMS 0,80% | 0,98 |
| | AMS 0,80% + AcOFe(III) 0,1% | <0,01 |
| X2CrNiMoN22-5-3 (1.4462) | APTS 0,5 % | 0,30 |
| | APTS 0,5 % + AcOFe(III) 0,1% | <0,01 |
| | AMS 0,28% | 0,29 |
| | AMS 0,28% + AcOFe(III) 0,1% | <0,01 |
| | AMS 0,80% | 1,62 |
| | AMS 0,80% + AcOFe(III) 0,1% | <0,01 |

Les valeurs des vitesses de corrosion avec de l'APTS 0,5 % et de l'AMS 0,28% en absence d'inhibiteur de corrosion acétate de fer (III) sont comparables (environ 0,3 mm/an), quelle que soit la nature du matériau AISI 316L ou type X2CrNiMoN22-5-3 (1.4462).

On constate que plus le milieu est concentré en AMS (0,8%), plus la vitesse de corrosion est grande en l'absence d'acétate de fer III.

En particulier dans le cas du matériau type X2CrNiMoN22-5-3 (1.4462), lorsque le milieu contient de l'AMS à 0,80 % et en l'absence d'inhibiteur acétate de fer (III), la vitesse de corrosion est très importante (1,62 mm/an).

Par contre, dès que l'on ajoute de l'acétate de fer (III), la vitesse de corrosion retombe à une valeur satisfaisante inférieure à 0,01 mm/an.

**Revendications**

1.  Procédé pour inhiber la corrosion des surfaces métalliques en contact avec un milieu d'estérification comprenant un acide carboxylique, un alcool, et un acide protonique fort, c'est-à-dire présentant un pKa dans l'eau inférieur à 2, comme catalyseur, **caractérisé par le fait que** l'on met en oeuvre, lors d'une réaction d'estérification entre ledit acide carboxylique et ledit alcool en présence dudit acide protonique fort, un carboxylate de fer (III).

2.  Procédé selon la revendication 1, **caractérisé par le fait que** le carboxylate de fer (III) est celui correspondant à l'acide carboxylique à estérifier, de préférence l'acétate de fer (III).

3.  Procédé selon la revendication 1 ou 2, **caractérisé par le fait que** le catalyseur acide protonique fort est un acide fort permettant une catalyse homogène choisi parmi l'acide sulfurique, les acides sulfoniques et leurs mélanges.

4.  Procédé selon la revendication 3, **caractérisé par le fait que** l'acide protonique fort est un acide sulfonique choisi parmi l'acide fluorosulfonique, l'acide chlorosulfonique ou l'acide trifluorométhanesulfonique, l'acide méthanesulfonique, l'acide éthanesulfonique, l'acide camphène-sulfonique, l'acide benzènesulfonique, les acides toluènesulfoniques, les acides xylènesulfoniques, les acides naphtalènesulfoniques, de préférence parmi l'acide para-toluènesulfonique ou l'acide méthanesulfonique.

5.  Procédé selon l'une des revendications 1 à 4, **caractérisé par le fait que** le catalyseur acide protonique fort est introduit à une concentration de 0,1 à 15 % en masse, de préférence de 0,2 à 10 % en masse et encore plus préférentiellement de 0,3 à 2 % en masse par rapport au milieu réactionnel.

6.  Procédé selon l'une des revendications 1 à 5, **caractérisé par le fait que** le carboxylate de fer (III) est introduit dans une concentration allant de 300 à 1500 ppm dans le milieu réactionnel, de préférence entre 400 et 1200 ppm.

**7.** Procédé selon l'une des revendications 1 à 6, **caractérisé par le fait que** l'acide carboxylique est choisi parmi les acides carboxyliques aliphatiques ayant de 1 à 6 atomes de carbone, de préférence l'acide carboxylique est l'acide acétique.

**8.** Procédé selon l'une des revendications 1 à 7, **caractérisé par le fait que** l'alcool est choisi parmi l'éthanol, le n-propanol, le butanol, et le cyclohexanol, de préférence l'alcool est l'éthanol.

**9.** Procédé selon l'une des revendications 1 à 8, **caractérisé par le fait que** les surfaces métalliques en contact avec le milieu d'estérification comprenant un acide carboxylique, un alcool et un catalyseur acide protonique fort sont des surfaces susceptibles de résister à la corrosion du milieu réactionnel choisies parmi alliages au nickel pouvant contenir du molybdène, du chrome, du cobalt, du fer, du cuivre, du manganèse, du titane, du zirconium, de l'aluminium, du carbone et du tungstène, de préférence les aciers inoxydables austénitiques ou austéno-ferritique.

**10.** Procédé selon l'une des revendications 1 à 9, **caractérisé par le fait que** la réaction d'estérification est mise en oeuvre dans un procédé en deux étapes : une réaction d'estérification suivie d'une opération de distillation.

**11.** Procédé selon la revendication 10, **caractérisé par le fait que** la réaction d'estérification est mise en oeuvre à une température comprise entre 50 et 150°C, de préférence entre 100 et 130°C et à une pression comprise entre 0,5 et 5 bars absolus.

**12.** Procédé selon l'une des revendications 1 à 9, **caractérisé par le fait que** la réaction d'estérification est mise en oeuvre dans un procédé en une étape, cette étape consistant en une opération de distillation réactive.

**13.** Utilisation d'un carboxylate de fer (III) comme inhibiteur de corrosion des surfaces métalliques en contact avec un milieu d'estérification comprenant un acide carboxylique, un alcool et un catalyseur acide protonique fort, c'est-à-dire présentant un pKa dans l'eau inférieur à 2.

**14.** Composition comprenant au moins un acide protonique fort choisi parmi l'acide para-toluènesulfonique ou l'acide méthanesulfonique et un carboxylate de fer (III).

**15.** Composition selon la revendication 14, **caractérisée par le fait que** le carboxylate de fer (III) est l'acétate de fer (III).

**Patentansprüche**

**1.** Verfahren zur Inhibierung der Korrosion von Metalloberflächen in Kontakt mit einem Veresterungsmedium, das eine Carbonsäure, einen Alkohol und eine starke Protonensäure, d. h. eine Protonensäure mit einem pKa-Wert in Wasser von weniger als 2, als Katalysator umfasst, **dadurch gekennzeichnet, dass** man bei einer Veresterungsreaktion zwischen der Carbonsäure und dem Alkohol in Gegenwart der starken Protonensäure ein Eisen(III)-carboxylat verwendet.

**2.** Verfahren nach Anspruch 1, **dadurch gekennzeichnet, dass** es sich bei dem Eisen(III)-carboxylat um dasjenige, das der zu veresternden Carbonsäure entspricht, vorzugsweise Eisen(III)-acetat, handelt.

**3.** Verfahren nach Anspruch 1 oder 2, **dadurch gekennzeichnet, dass** es sich bei der als Katalysator dienenden starken Protonensäure um eine starke Säure handelt, die eine homogene Katalyse ermöglicht und aus Schwefelsäure und Sulfonsäuren und Mischungen davon ausgewählt wird.

**4.** Verfahren nach Anspruch 3, **dadurch gekennzeichnet, dass** es sich bei der starken Protonensäure um eine Sulfonsäure handelt, die aus Fluorsulfonsäure, Chlorsulfonsäure, Trifluormethansulfonsäure, Methansulfonsäure, Ethansulfonsäure, Camphensulfonsäure, Benzolsulfonsäure, Toluolsulfonsäuren, Xylolsulfonsäuren und Naphthalinsulfonsäuren, vorzugsweise aus para-Toluolsulfonsäure und Methansulfonsäure, ausgewählt wird.

**5.** Verfahren nach einem der Ansprüche 1 bis 4, **dadurch gekennzeichnet, dass** die als Katalysator dienende starke Protonensäure in einer Konzentration von 0,1 bis 15 Gew.-%, vorzugsweise von 0,2 bis 10 Ges.-% und noch weiter bevorzugt von 0,3 bis 2 Ges.-%, bezogen auf das Reaktionsmedium, eingetragen wird.

**6.** Verfahren nach einem der Ansprüche 1 bis 5, **dadurch gekennzeichnet, dass** das Eisen(III)-carboxylat in einer Konzentration im Bereich von 300 bis 1500 ppm und vorzugsweise zwischen 400 und 1200 ppm in das Reaktionsmedium eingetragen wird.

7. Verfahren nach einem der Ansprüche 1 bis 6, **dadurch gekennzeichnet, dass** die Carbonsäure aus aliphatischen Carbonsäuren mit 1 bis 6 Kohlenstoffatomen ausgewählt wird und es sich bei der Carbonsäure vorzugsweise um Essigsäure handelt.

8. Verfahren nach einem der Ansprüche 1 bis 7, **dadurch gekennzeichnet, dass** der Alkohol aus Ethanol, n-Propanol, Butanol und Cyclohexanol ausgewählt wird und es sich bei dem Alkohol vorzugsweise um Ethanol handelt.

9. Verfahren nach einem der Ansprüche 1 bis 8, **dadurch gekennzeichnet, dass** es sich bei den Metalloberflächen in Kontakt mit dem Veresterungsmedium, das eine Carbonsäure, einen Alkohol und eine als Katalysator dienende starke Protonensäure umfasst, um Oberflächen handelt, die der Korrosion durch das Reaktionsmedium widerstehen können und aus Nickellegierungen, die Molybdän, Chrom, Cobalt, Eisen, Kupfer, Mangan, Titan, Zirkonium, Aluminium, Kohlenstoff und Wolfram enthalten können, vorzugsweise austenitischen oder austenitisch-ferritischen nichtrostenden Stählen, ausgewählt werden.

10. Verfahren nach einem der Ansprüche 1 bis 9, **dadurch gekennzeichnet, dass** die Veresterungsreaktion in einem zweischrittigen Verfahren durchgeführt wird: einer Veresterungsreaktion mit nachfolgender Destillation.

11. Verfahren nach Anspruch 10, **dadurch gekennzeichnet, dass** die Veresterungsreaktion bei einer Temperatur zwischen 50 und 150 °C, vorzugsweise zwischen 100 und 130 °C, und einem Druck zwischen 0,5 und 5 bar absolut durchgeführt wird.

12. Verfahren nach einem der Ansprüche 1 bis 9, **dadurch gekennzeichnet, dass** die Veresterungsreaktion in einem einschrittigen Verfahren durchgeführt wird, wobei dieser Schritt aus einer Reaktivdestillation besteht.

13. Verwendung eines Eisen(III)-carboxylats als Inhibitor der Korrosion von Metalloberflächen in Kontakt mit einem Veresterungsmedium, das eine Carbonsäure, einen Alkohol und eine starke Protonensäure, d. h. eine Protonensäure mit einem pKa-Wert in Wasser von weniger als 2, als Katalysator umfasst.

14. Zusammensetzung, die mindestens eine starke Protonensäure, die aus para-Toluolsulfonsäure und Methansulfonsäure ausgewählt ist, und ein Eisen(III)-carboxylat umfasst.

15. Zusammensetzung nach Anspruch 14, **dadurch gekennzeichnet, dass** es sich bei dem Eisen(III)-carboxylat um Eisen(III)-acetat handelt.

**Claims**

1. Process for inhibiting the corrosion of the metal surfaces in contact with an esterification medium comprising a carboxylic acid, an alcohol and a strong protonic acid, i.e. with a pka in water of less than 2, as catalyst, **characterized in that** an iron(III) carboxylate is used in an esterification reaction between said carboxylic acid and said alcohol in the presence of said strong protonic acid.

2. Process according to Claim 1, **characterized in that** the iron(III) carboxylate is that corresponding to the carboxylic acid to be esterified, preferably iron(III) acetate.

3. Process according to Claim 1 or 2, **characterized in that** the strong protonic acid catalyst is a strong acid enabling homogeneous catalysis chosen from sulfuric acid and sulfonic acids, and mixtures thereof.

4. Process according to Claim 3, **characterized in that** the strong protonic acid is a sulfonic acid chosen from fluorosulfonic acid, chlorosulfonic acid or trifluoromethanesulfonic acid, methanesulfonic acid, ethanesulfonic acid, camphenesulfonic acid, benzenesulfonic acid, toluenesulfonic acids, xylenesulfonic acids and naphthalenesulfonic acids, preferably from para-toluenesulfonic acid and methanesulfonic acid.

5. Process according to one of Claims 1 to 4, **characterized in that** the strong protonic acid catalyst is introduced at a concentration of from 0.1% to 15% by mass, preferably from 0.2% to 10% by mass and even more preferentially from 0.3% to 2% by mass relative to the reaction medium.

6. Process according to one of Claims 1 to 5, **characterized in that** the iron(III) carboxylate is introduced in a concentration ranging from 300 to 1500 ppm and preferably between 400 and 1200 ppm into the reaction medium.

7. Process according to one of Claims 1 to 6, **characterized in that** the carboxylic acid is chosen from aliphatic carboxylic acids containing from 1 to 6 carbon atoms; preferably, the carboxylic acid is acetic acid.

8. Process according to one of Claims 1 to 7, **characterized in that** the alcohol is chosen from ethanol, n-propanol, butanol and cyclohexanol, and preferably the alcohol is ethanol.

9. Process according to one of Claims 1 to 8, **characterized in that** the metal surfaces in contact with the esterification medium comprising a carboxylic acid, an alcohol and a strong protonic acid catalyst are surfaces that are capable of withstanding corrosion by the reaction medium, chosen from nickel alloys that may contain molybdenum, chromium, cobalt, iron, copper, manganese, titanium, zirconium, aluminum, carbon and tungsten, preferably austenitic or austeno-ferritic stainless steels.

10. Process according to one of Claims 1 to 9, **characterized in that** the esterification reaction is performed in a two-step process: an esterification reaction followed by a distillation operation.

11. Process according to Claim 10, **characterized in that** the esterification reaction is performed at a temperature of between 50 and 150°C and preferably between 100 and 130°C and at a pressure of between 0.5 and 5 bar absolute.

12. Process according to one of Claims 1 to 9, **characterized in that** the esterification reaction is performed in a one-step process, this step consisting of a reactive distillation operation.

13. Use of an iron(III) carboxylate as a corrosion inhibitor for the metal surfaces in contact with an esterification medium comprising a carboxylic acid, an alcohol and a strong protonic acid catalyst, i.e. with a pka in water of less than 2.

14. Composition comprising at least one strong protonic acid chosen from para-toluenesulfonic acid and methanesulfonic acid and an iron(III) carboxylate.

15. Composition according to Claim 14, **characterized in that** the iron(III) carboxylate is iron(III) acetate.

**EP 2 576 493 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- GB 1173089 A
- EP 1220829 A
- EP 0158499 A
- EP 2060555 A
- WO 2007099071 A

### Littérature non-brevet citée dans la description

- **ROBERT H. PERRY et al.** Perry's Chemical Engineers' Handbook. 1984, 23-44